# EUROPÄISCHE PATENTANMELDUNG

(11) **EP 4 148 447 A1**
(43) Veröffentlichungstag der Anmeldung: **15.03.2023**
(21) Anmeldenummer: 21195956.4
(22) Anmeldetag: 10.09.2021
(51) Int. Cl.: G01R 33/36, A61B 5/11, G01R 33/565, G01R 33/567, A61B 5/055, A61B 5/00

(54) **PILOTTON-SIGNAL-GENERATOR, MAGNETRESONANZTOMOGRAPH, VERFAHREN ZUM ÜBERTRAGEN EINES SYNCHRONISIERUNGSSIGNALS UND COMPUTERPROGRAMMPRODUKT**

(71) Anmelder: Siemens Healthcare GmbH, 91052 Erlangen (DE)
(72) Erfinder: Bacher, Mario, 91052 Erlangen (DE); Bollenbeck, Jan, 91330 Eggolsheim (DE); Speier, Peter, 91056 Erlangen (DE)

(57) **Zusammenfassung**

Die Erfindung betrifft einen Pilotton-Signal-Generator, einen Magnetresonanztomograph, ein Verfahren zum Übertragen eines Synchronisierungssignals und ein Computerprogrammprodukt. Der Pilotton-Signal-Generator umfasst eine Empfangseinheit zum Empfang eines Synchronisierungssignals einer Systemsteuereinheit eines Magnetresonanztomographen. Dabei umfasst das Synchronisierungssignals ein Taktsignal, und der Pilotton-Signal-Generator ist ausgebildet ist, ein Pilotton-Signal in Abhängigkeit von dem Synchronisierungssignal auszusenden.

## Beschreibung

Die Erfindung betrifft einen Pilotton-Signal-Generator, einen Magnetresonanztomograph, ein Verfahren zum Übertragen eines Synchronisierungssignals und ein Computerprogrammprodukt.

Magnetresonanztomographen (MRT) sind bildgebende Vorrichtungen, die zur Abbildung eines Untersuchungsobjektes, z.B. eines menschlichen oder tierischen Patienten, Kernspins des Untersuchungsobjektes mit einem starken äußeren Magnetfeld (B₀-Feld) ausrichten und durch ein magnetisches Wechselfeld (B₁-Feld) zur Präzession um diese Ausrichtung anregen. Die Präzession bzw. Rückkehr der Kernspins aus diesem angeregten Zustand in einen Zustand mit geringerer Energie wiederum erzeugt als Antwort ein Magnetresonanzsignal in Form eines magnetisches Wechselfelds, das über Empfangsspulen empfangen werden kann. Zum Empfang dieses Magnetresonanzsignals werden vorzugsweise lokale Empfangsspulen, sogenannte Lokalspulen verwendet, die zur Erzielung eines besseren Signal-Rauschverhältnisses (SNR) unmittelbar am Untersuchungsobjekt angeordnet werden.

Mit Hilfe von magnetischen Gradientenfeldern wird den Signalen eine Ortskodierung aufgeprägt, die nachfolgend eine Zuordnung von dem empfangenen Magnetresonanzsignal zu einem Volumenelement ermöglicht. Das empfangene Magnetresonanzsignal wird dann üblicherweise rekonstruiert, um eine oder mehrere Magnetresonanzabbildungen zu erstellen.

Die Druckschrift US 10222443 B2 offenbart ein Verfahren, welches es ermöglicht, zeitliche Abläufe während einer Magnetresonanzuntersuchung auf physiologische Bewegungen, wie z.B. Atmung und/oder Herzschlag, zu triggern. Hierdurch können Bewegungsartefakte vermieden (prospektive Bewegungskorrektur) und/oder im Zuge einer digitalen Nachverarbeitung beseitigt werden (retrospektive Bewegungskorrektur). Es wird vorgeschlagen, während der Magnetresonanzuntersuchung ein schwaches, hinreichend konstantes, insbesondere hinsichtlich Amplitude und/oder Frequenz, hochfrequentes (HF) Pilotton-Signal derart auszusenden, dass dieses über eine oder mehrere Empfangsspulen empfangen werden kann, ohne den Empfang des Magnetresonanzsignals zu stören. Die Detektion von Patientenbewegungen geschieht über die Auswertung des zeitlichen Verlaufs von Amplitude und/oder Phase des empfangenen Pilotton-Signals. Ein Pilotton-Signal-Generator zum Aussenden eines Pilotton-Signals ist beispielhaft in der Druckschrift US 10393845 B2 beschrieben.

Als Aufgabe der vorliegenden Erfindung kann gesehen werden, einen Pilotton-Signal-Generator bereitzustellen, der eine verbesserte Bewegungskorrektur ermöglicht. Insbesondere ist wünschenswert, eine exaktere Auswertung des Pilotton-Signals zu ermöglichen.

Die Aufgabe wird durch die Merkmale der unabhängigen Ansprüche gelöst. Vorteilhafte Ausgestaltungen sind in den abhängigen Ansprüchen beschrieben.

Demnach wird ein Pilotton-Signal-Generator vorgeschlagen, wobei der Pilotton-Signal-Generator eine Empfangseinheit zum Empfang eines Synchronisierungssignals einer Systemsteuereinheit eines Magnetresonanztomographen umfasst. Dabei umfasst das Synchronisierungssignals ein Taktsignal, und der Pilotton-Signal-Generator ist ausgebildet ist, ein Pilotton-Signal in Abhängigkeit von dem Synchronisierungssignal auszusenden.

Der Pilotton-Signal-Generator ist vorzugsweise ausgelegt, ein das Pilotton-Signal als elektromagnetisches Hochfrequenzsignal in ein Untersuchungsobjekt auszusenden. Ein Pilotton-Signal-Empfänger, insbesondere eine Lokalspule und/oder eine fest in den Magnetresonanztomographen eingebaute Körperspule, ist vorzugsweise ausgelegt, das Pilotton-Signal zu empfangen und zur Auswertung einer Information über einen physiologischen Vorgang in dem Untersuchungsobjekt und/oder einer Bewegung des Untersuchungsobjekts an eine Auswerteeinheit zu übertragen.

Der Pilotton-Signal-Generator kann beispielsweise Teil einer Lokalspule sein, insbesondere in einer Lokalspule integriert und/oder eingebaut sein. Vorteilhafterweise kann dadurch der Pilotton-Signal-Generator besonders nahe am Untersuchungsobjekt positioniert werden, so dass vorteilhafterweise ein nach dem Aussenden durch den Pilotton-Signal-Generator wieder, beispielsweise durch Empfangsantennen der Lokalspule, empfangenes Pilotton-Signal ein besonders hohes SNR aufweist.

Das generierte Pilotton-Signal weist vorzugsweise ein erstes Frequenzband auf, wobei der Pilotton-Signal-Empfänger, insbesondere der Pilotton-Signal-Empfänger, dazu ausgebildet ist, ein Empfangs-Frequenzband aufzunehmen, das das erste Frequenzband umfasst. Vorzugsweise umfasst der Magnetresonanztomograph eine Hochfrequenzeinheit, die ausgebildet ist, einen Hochfrequenz-Puls mit einem zweiten Frequenzband auszugegeben, wobei ein Magnetresonanzsignal des Hochfrequenz-Pulses mit der Pilotton-Signal-Empfänger erfasst wird, wobei das Magnetresonanzsignal ein drittes Frequenzband aufweist, das zumindest im Wesentlichen außerhalb des ersten Frequenzbandes liegt. Vorteilhafterweise kollidiert das erste Frequenzband nicht mit dem eigentlichen Messsignal, dem Magnetresonanzsignal, das im dritten Frequenzband liegt.

Das Taktsignal ist vorzugsweise ein Taktsignal, insbesondere ein Referenztaktsignal, des Magnetresonanztomographen. Vorzugsweise ist das Taktsignal geeignet ist, einen Referenztakt des Magnetresonanztomographen zu übertragen. Vorteilhafterweise wird das Pilot-Signal phasensynchron an den Referenztakt des Magnetresonanztomographen angebunden. Vorteilhafterweise kann dadurch vermieden werden, dass die absolute Phase des Pilot-Signal unkontrolliert schwankt. Die absolute Phase kann somit ausgewertet werden. Eine etwaige Beschränkung der Phasenauswertung auf die relativen Phasen zwischen Empfangskanälen kann dadurch überwunden werden. Stattdessen kann eine Phaseninformation eines Empfangskanals oder einer Kanalkombination, die als Referenz dient, ausgewertet werden. Ferner können SNR-Verluste in der Pilotton-Phase durch Differenzbildung von mehreren Messsignalen vermieden werden.

Je nach Detektion des Pilot-Signal übersetzen sich Phasenschwankungen bei Tiefpass-Filterung in Amplitudenschwankungen. Durch eine Synchronisierung mittels des Synchronisierungssignals können Phasenschwankungen reduziert werden, so dass auch die empfangene Amplitude des Pilot-Signals stabiler wird.

Eine Ausführungsform des Pilotton-Signal-Generators sieht vor, dass das Synchronisierungssignal ein optisches Datensignal ist, wobei die Empfangseinheit einen ersten Sensor zum Empfang des optischen Datensignals aus der Umgebung des Pilotton-Signal-Generators umfasst und ausgebildet ist, aus dem optischen Datensignal ein elektrisches Sensorsignal zu erzeugen.

Vorzugsweise ist das optisches Datensignal nicht nur ein Signal in dem vom menschlichen Auge üblicherweise sichtbaren Wellenlängenbereich von 380 nm bis 750 nm, sondern auch benachbarten Wellenlängenbereichen wie Ultraviolett zwischen 150 nm und 380 nm oder nahes Infrarot zwischen 750 nm und 2000 nm. Insbesondere weist das optische Datensignal eine Wellenlänge zwischen 780 nm und 1000 nm auf. Insbesondere ist die Energie der Lichtquanten größer als 0,8 eV.

Als Umgebung wird dabei der den Pilotton-Signal-Generator umgebende freie Raum angesehen, insbesondere in einer anwendungsgemäßen Position, beispielsweise in einem Patiententunnel. Insbesondere wird darunter keine geführte optische Verbindung wie ein Lichtwellenleiter zwischen Pilotton-Signal-Generator und Magnetresonanztomograph verstanden.

Vorzugsweise wandelt der erste Sensor das optische Datensignal in ein elektrisches Signal um, welches weiter in dem Pilotton-Signal-Generator zur Erzeugung eines Pilotton-Signals genutzt wird. Das optische Datensignal überträgt dabei vorzugsweise eine Information von dem Magnetresonanztomographen zu dem Pilotton-Signal-Generator. Der Sensor kann beispielsweise eine Foto-Diode, ein Fototransistor oder ein anderes elektronisches Element als Detektorelement aufweisen oder eine Kombination elektronischer Bauelemente sein, beispielsweise mit einem Verstärker, das bzw. die ein optisches Signal in ein elektrisches Signal bzw. Sensorsignal wandelt.

Auf vorteilhafte Weise erlaubt der drahtlose Pilotton-Signal-Generator über den optischen Sensor eine Übertragung von Information zur Steuerung des Pilotton-Signal-Generators ohne Störung des Magnetresonanzempfangs oder beim Betrieb des Magnetresonanztomographen störende Kabel.

Eine weitere Ausführungsform des Pilotton-Signal-Generators sieht vor, dass die Empfangseinheit einen zu dem ersten Sensor benachbarten zweiten Sensor umfasst, wobei der erste Sensor ausgelegt ist, aus dem optischen Datensignal ein erstes Ausgangssignal zu erzeugen und der zweite Sensor ausgelegt ist, aus dem gleichen optischen Datensignal ein zweites Ausgangssignal zu erzeugen, wobei das erste Ausgangssignal eine zu dem zweiten Ausgangssignal inverse Amplitude aufweist, wobei die Pilotton-Signal-Generator einen Inverter aufweist, der das Ausgangssignal des ersten Sensors invertiert, und ein Summierglied, das ausgelegt ist, das invertierte Ausgangssignal des ersten Sensors und das Ausgangssignal des zweiten Sensors zu dem Sensorsignal zu addieren.

Als benachbart kann dabei insbesondere ein Abstand zwischen dem ersten Sensor und dem zweiten Sensor angesehen, in dem die von dem Magnetresonanztomographen erzeugten elektrischen und/oder magnetischen Felder im Wesentlich gleiche Stärke aufweisen, sodass von diesen Feldern verursachte Störungen in dem ersten und dem zweiten Sensor im Wesentlichen identisch sind. Der Abstand kann beispielsweise kleiner als 2 mm, 5 mm, 1 cm oder 5 cm sein. Vorzugsweise sind dabei die Sensoren so angeordnet bzw. ausgerichtet, dass eine optische Signalquelle in einer Umgebung des Pilotton-Signal-Generators in einem Detektorelement des ersten Sensors und des zweiten Sensors eine im Wesentlichen gleiche Wirkung, insbesondere in Bezug auf Stärke bzw. Betrag erzielt, beispielsweise eine annähernd gleiche Anzahl von Elektronen-Loch-Paaren in den beiden Sensoren erzeugen.

Vorzugsweise ist der erste Sensor ausgelegt, aus dem optischen Datensignal ein erstes Ausgangssignal zu erzeugen und der zweite Sensor ist ausgelegt, aus dem gleichen optischen Datensignal ein zweites Ausgangssignal zu erzeugen. Das erste Ausgangssignal weist vorzugsweise eine zu dem zweiten Ausgangssignal invertierte Amplitude auf, mit anderen Worten, der Betrag ist im Wesentlichen gleich, das Vorzeichen aber unterschiedlich. Dabei wird das Ausgangssignal in Bezug auf bzw. relativ zu einem Ruhepegel bzw. Offset betrachtet, den der erste Sensor und der zweite Sensor beispielsweise ohne Einwirkung eines optischen Signals erzeugt, oder als Wechselstromanteil eines von den Sensoren erzeugten Signals mit Frequenzanteilen größer 1 Hz, 100 Hz, 1 kHz, 100 kHz oder 1 MHz. Entgegengesetzte Vorzeichen können beispielsweise erreicht werden, indem eine Foto-Diode im ersten Sensor an die positive Versorgungsspannung angeschlossen ist und über einen Widerstand mit der negativen Versorgungsspannung, während eine Foto-Diode im zweiten Sensor an die negative Versorgungsspannung angeschlossen ist und über einen Widerstand mit der positiven Versorgungsspannung verbunden ist. Das Ausgangssignal mit unterschiedlichen Vorzeichen liegt dann jeweils an den Verbindungspunkten der Foto-Dioden mit den Widerständen an.

Vorzugsweise weist der Pilotton-Signal-Generator einen Inverter auf, der das Ausgangssignal des ersten Sensors invertiert, und ein Summierglied, das ausgelegt ist, das invertierte Ausgangssignal des ersten Sensors und das Ausgangssignal des zweiten Sensors zu einem Sensorsignal zu addieren.

Ein Invertieren des Signals kann beispielsweise durch eine Emitterschaltung mit einem Transistor erreicht werden.

Auf vorteilhafte Weise werden durch optische Signale erzeugte Ausgangssignale durch das inverse Vorzeichen und die anschließende Invertierung mit gleichem Vorzeichen addiert und so verstärkt, während elektrisch und/oder magnetisch induzierte Störungen durch die einfache Invertierung sich bei der Summation idealerweise aufheben, sodass in dem Sensorsignal der Störanteil wesentlich reduziert ist.

Vorzugsweise weist das Synchronisierungssignal, insbesondere das Taktsignal, eine Amplitudenmodulation auf, wobei der Pilotton-Signal-Generator ausgebildet ist, das optisches Datensignal zu demodulieren.

Vorzugsweise umfasst der Pilotton-Signal-Generators ein Filter auf, das ausgelegt ist, eine Trägersignalfrequenz bzw. eine erste Modulationsfrequenz des Sensorsignals zu selektieren. Mit anderen Worten, der Filter weist ein lokales oder vorzugsweise auch globales Minimum einer Durchlassdämpfung für eine vorbestimmte Trägersignalfrequenz auf. Vorzugsweise dämpft das Filter ein Sensorsignal mit einer Frequenz in einem Abstand von einer Oktave bzw. bei einer Frequenz gleich der halben bzw. doppelten Trägersignal-frequenz gegenüber Dämpfung bei der Modulationsfrequenz um mehr als 24 dB, 30 dB oder 36 dB. Das Filter kann vorzugsweise als Bandpassfilter ausgelegt sein, aber beispielsweise auch als Tiefpassfilter, je nach Frequenzspektrum der Modulation des optischen Signals.

Eine weitere Ausführung der Pilotton-Signal-Generator sieht vor, dass der der Pilotton-Signal-Generator einen, insbesondere schmalbandigen, Phase-Lock-Loop-Schaltkreis (PPL-Schaltkreis) zur Synchronisation des Pilotton-Signals umfasst, wobei ein aus dem Sensorsignal abgeleitetes Signal als Referenzsignal für den Phase-Lock-Loop-Schaltkreis dient.

Eine weitere Ausführungsform des Pilotton-Signal-Generators sieht vor, dass der Pilotton-Signal-Generator ein Filter aufweist, das ausgelegt ist, eine Modulationsfrequenz des Sensorsignals zu selektieren, wobei der, insbesondere schmalbandige, PLL-Schaltkreis ausgelegt ist, einen Oszillator in Abhängigkeit von der Modulationsfrequenz, und vorzugsweise der Modulationsphase, zu stabilisieren.

Insbesondere ist der, insbesondere schmalbandige, PLL-Schaltkreis ausgelegt, einen Oszillator, vorzugsweise einen Quarz-Oszillator und/oder einen spannungsgesteuerten Oszillator (engl. voltage controlled oscillator, VCO), in Abhängigkeit von der Trägersignalfrequenz zu stabilisieren. Der Quarz-Oszillator kann insbesondere spannungsgesteuerter Quarzoszillator (engl. voltage controlled crystal oscillator, VCXO) sein. Bei spannungsgesteuerten Oszillatoren kann vorteilhafterweise eine Oszillatorfrequenz durch Anlegen einer elektrischen Spannung verändert werden kann. Ein schmalbandiger PLL-Schaltkreis ist hier insbesondere ein PLL-Schaltkreis, der bei einer Frequenzabweichung von der Oszillator-Eigenfrequenz kleiner als 100ppm, 10ppm oder 1ppm einrastet. Vorteilhafterweise ist ein schmalbandiger PLL-Schaltkreis störungssicher und kann auch bei kurzen Aussetzern des Sensorsignals ein genaues Taktsignal bereitstellen.

Eine weitere Ausführungsform des Pilotton-Signal-Generators sieht vor, dass der Pilotton-Signal-Generator ein Filter umfasst, das ausgelegt ist, eine ungeradzahlige Harmonische eines Ausgangssignals des PLL-Schaltkreises zu selektieren. Weist beispielsweise das Ausgangssignals des PLL-Schaltkreises eine Frequenz von 12.5 MHz auf, dann kann die selektierte Harmonische insbesondere die fünfte Harmonische sein, die eine Frequenz von 62.5 Mhz (gleich 5 mal 12.5 Mhz) aufweist.

Insbesondere weist der Oszillator, insbesondere der VCXO, einen rechteckförmigen Zeitverlauf auf. Das Signalspektrum enthält somit vornehmlich Harmonische ungeradzahliger Ordnung.

Damit eigenen sich solche Oszillatoren besonders gut, um eine ungeradzahlige Harmonische zu selektieren. Über ein BandpassFilter kann beispielsweise hiervon eine ungeradzahlige Harmonische, beispielsweise die fünfte, selektiert werden und einer Sendeantenne, insbesondere einer kleinen Leiterschleife, des Pilotton-Signal-Generators zum Aussenden zugeführt werden. Das hierüber erzeugte magnetische Wechselfeld koppelt beispielsweise in die Empfangsantennen, beispielsweise in Lokalspulen angeordnete Empfangsantennen, des Magnetresonanztomographen.

In einer möglichen Ausführungsform des Pilotton-Signal-Generators weist der Pilotton-Signal-Generator einen Amplitudendemodulator mit einem Kompensationskreis auf. Der Kompensationskreis ist ausgelegt, einen im Vergleich zu einer Modulationsfrequenz des Modulationssignals niederfrequenten Signalanteil des Sensorsignals zu kompensieren. Als niederfrequenter Anteil im Sinne der Erfindung kann ein spektraler Anteil des von dem Amplitudendemodulator demodulierten Signals angesehen werden, dessen Frequenz kleiner als 10%, 1% oder ein Promille der Modulationsfrequenz ist. Mit anderen Worten, dessen Frequenz kleiner als 10%, 1% oder ein Promille des zu übertragenden Nutzsignals, z.B. des Synchronisierungssignals, insbesondere des Taktsignals und/oder Steuersignals. Insbesondere können Anteile als niederfrequent angesehen werden, die durch Bewegungen der Pilotton-Signal-Generators (beispielsweise durch Bewegungen einer Lokalspule, in der der Pilotton-Signal-Generator angeordnet ist) bei der Anwendung oder durch externe Lichtquellen verursacht werden und im Bereich unter 200 Hz, 120 Hz oder 60 Hz sind.

Intensitätsänderungen durch Bewegung oder Abschattung sind üblicherweise langsame Signaländerungen, die aufgrund dieses Frequenzunterschiedes vorteilhafterweise von einem Kompensationskreis von einer Taktfrequenz oder einem Datensignal getrennt und/oder unterdrückt werden können.

In einer möglichen Ausführungsform des Pilotton-Signal-Generators umfasst der Kompensationskreis einen Komparator mit einem Referenzspannungseingang, der über einen Tiefpass in erster Signalverbindung mit dem Sensorsignal steht. Insbesondere weist der Kompensationskreis einen als Komparator verschalteten Differenzverstärker mit einem Referenzspannungseingang auf, der über ein Dämpfungsglied, vorzugsweise in Form eines resistiven Spannungsteilers, in einer Reihenschaltung mit einem Tiefpass in erster Signalverbindung mit dem Sensorsignal steht. Als Komparator kann dabei eine Schaltung angesehen werden, die zwei Eingangssignale vergleicht und bereits bei einer geringen Differenz der Eingangssignale ein Vergleichsergebnis in Form einer durchgesteuerten Ausgangsspannung bereitstellt. Der Komparator entspricht insofern einem Differenzverstärker mit einer hohen Verstärkung. Es ist dabei beispielsweise denkbar, dass der Referenzspannungseingang nichtinvertierend ist, und am invertierenden Eingang des Differenzverstärkers das Sensorsignal oder ein dazu proportionales Signal anliegt.

Über den Tiefpass werden so nur die niederfrequenten Anteile im Sensorsignal als Komparator-Referenzsignal an den nichtinvertierenden Eingang des Differenzverstärkers angelegt. Hierdurch folgt das Komparator-Referenz-Signal auf vorteilhafte Weise langsamen Änderungen, wie sie z.B. durch Bewegung oder Abschattung entstehen. Vorzugsweise reduziert ein vorgeschalteter Spannungsteiler die Spannung des tiefpassgefilterten Referenzsignals, sodass diese ohne Steuersignalübertragung unterhalb der Sensorspannung liegt und das Komparator-Ausgangssignal ausschließlich auf den durch das sich schnell ändernde Modulations-Signal hervorgerufenen Anteil im Sensorsignal reagiert.

In einer denkbaren Ausführungsform des Pilotton-Signal-Generators weist die erste Signalverbindung ein Track-und-Hold-Glied auf, das in Abhängigkeit von einer Differenzspannung zwischen Sensorsignal und Referenzspannung betätigt wird. Beispielsweise kann ein elektronischer Schalter zwischen einer Quelle des Sensorsignals und dem Tiefpass angeordnet sein.

Auf vorteilhafte Weise trennt dann der Schalter den Sensorsignalpfad zum Tiefpass auf, wenn eine Sensorsignalspannung die Referenzspannung aufgrund der schnellen Modulation unterschreitet, wodurch die Referenzspannung solange konstant gehalten wird, bis die Sensorsignalspannung aufgrund des Modulationsinhalts die Referenzspannung wieder überschreitet.

Eine mögliche Ausführungsform des Pilotton-Signal-Generators sieht vor, dass das Synchronisierungssignal ein Steuersignal umfasst, wobei der Pilotton-Signal-Generator eine Steuerkomponente umfasst, die geeignet ist, anhand des Steuersignals eine Amplitude und/oder Phase des Pilotton-Signals zu steuern. Vorteilhafterweise ist das Steuersignal geeignet, Steuerbefehle zur Erzeugung des Pilotton-Signals zu übertragen. Beispielsweise könnte zur Detektion einer schnellen Bewegung, z.B. einer Herzbewegung, mit hoher Amplitude gesendet werden, zur Detektion einer langsamen Bewegung, z.B. einer Atembewegung, mit einer niedrigen, und bei Messungen die bewegungsunempfindlich sind, könnte das Pilotton-Signal abschaltet werden.

Eine mögliche Ausführungsform des Pilotton-Signal-Generators sieht vor, dass die Steuerkomponente des Pilotton-Signal-Generators ferner geeignet ist, anhand des Steuersignals eine für den Pilotton-Signal-Generator generatorspezifische Information zu ermitteln, um den Pilotton-Signal-Generator (insbesondere gegenüber etwaigen anderen Pilotton-Signal-Generatoren, die an, in und/oder um den Magnetresonanztomographen angeordnet sind) spezifisch zu steuern. Insbesondere kann den jeweiligen Pilotton-Signal eine spezifische Phase und/oder Amplitude aufgeprägt werden. Vorteilhafterweise können die danach empfangenen Pilotton-Signale anhand dieser aufgeprägten Eigenschaften separiert werden, beispielsweise durch eine Auswerteeinheit des Magnetresonanztomographen.

Eine mögliche Ausführungsform des Pilotton-Signal-Generators sieht vor, dass der Pilotton-Signal-Generator eine Sendeantenne umfasst, und der der Pilotton-Signal-Generator ausgelegt ist, über die Sendeantenne ein Pilotton-Signal auszusenden. Insbesondere kann der Pilotton-Signal-Generator ein Entkopplungselement umfassen zur Entkopplung eines Senderausgangs von Signalen, die die Antenne in einem Magnetresonanztomographen durch Anregungspulse des Magnetresonanztomographen empfängt. Beispielsweise umfasst das Entkopplungselement eine Diode oder ein anderes Bauelement mit nichtlinearer Kennlinie und/oder ein Element mit frequenzabhängiger Kennlinie.

In einem Aufnahmebereich des Magnetresonanztomographen, in den sich das Untersuchungsobjekt während einer Magnetresonanzmessung befindet, werden zur Anregung der Kernspins Hochfrequenzfelder mit einer Leistung üblicherweise im Bereich von Kilowatt erzeugt, die eine ungeschützte Elektronik des Pilotton-Signal-Generators zerstören kann, insbesondere wenn dies zwangsläufig mit einer Antenne verbunden ist, die dem externen Hochfrequenzfeld ausgesetzt ist. Auf vorteilhafte Weise sorgt das Entkopplungselement dafür, dass der Ausgang des Pilotton-Signal-Generators von über die Antenne eingestrahlten Anregungspulsen entkoppelt ist und nicht durch diese zerstört werden kann.

Ferner wird ein Magnetresonanztomograph zur Untersuchung eines Untersuchungsobjekts. Der Magnetresonanztomograph umfasst einen optischen Sender aufweist, ausgelegt ist, ein optisches Datensignal mittels einer optischen Freiluftübertragung an zumindest einen Pilotton-Signal-Generator zu übertragen. Vorzugsweise ist der optische Sender ausgelegt, das optische Datensignal in den Patiententummelt z strahlen und/oder durch Streuung an einer Oberfläche des Patiententunnels zu verteilen.

Das optische Datensignal kann beispielsweise ein Synchronisierungssignal sein. Das Synchronisierungssignal kann insbesondere ein Taktsignal und/oder ein Steuersignal aufweisen.

Vorzugsweise umfasst der Magnetresonanztomograph zumindest eine Empfangsspule umfasst, wobei die zumindest eine Empfangsspule ausgebildet ist, durch den zumindest einen Pilotton-Signal-Generator ausgesendete Pilotton-Signale zu empfangen. Vorteilhafterweise ist die zumindest eine Empfangsspule zudem auch ausgebildet, Magnetresonanzsignale empfangen, so dass zum Empfang der Pilotton-Signale und der Magnetresonanzsignale nur eine Empfangsspule notwendig ist.

Vorzugsweise umfasst der Magnetresonanztomograph zumindest eine Auswerteeinheit zur Auswertung der durch die Empfangsspule empfangenen Pilotton-Signale, wobei die Auswerteeinheit ausgebildet ist, anhand der durch die Empfangsspule empfangenen Pilotton-Signale eine Bewegung des Untersuchungsobjekts zu ermitteln. Vorteilhafterweise kann damit eine Korrektur der Bewegung durchgeführt, welche zu einer besseren Qualität etwaiger Magnetresonanzabbildungen führt.

Vorzugsweise ist die Auswerteeinheit ausgebildet, empfangene Pilotton-Signale mehrerer Pilotton-Signal-Generatoren zu separieren und insbesondere ganz oder teilweise einem von mehreren Pilotton-Signale sendenden Pilotton-Signal-Generatoren zuzuordnen. Vorteilhafterweise können dadurch die verschiedenen Pilotton-Signal-Generatoren individuell gesteuert werden.

In einer möglichen Ausführungsform des Magnetresonanztomographen weist der optische Sender einen Amplitudenmodulator auf. Vorzugsweise ist der Amplitudenmodulator ausgelegt, eine Lichtintensitätsänderung zur Modulation des optischen Signals für die Übertragung des optischen Signals, insbesondere des Synchronisierungssignal, synchron auszuführen. Beispielsweise könnte der Amplitudenmodulator ein Sample-and-Hold-Glied oder Flip-Flop aufweisen, das eine Änderung des Steuersignals nur mit einer Flanke des Taktsignals auf einen Multiplizierer des Amplitudenmodulators schaltet. Insbesondere wird hier unter dem Amplitudenmodulator eine Vorrichtung verstanden, die ausgelegt ist, bei dem optischen Signal auch Zwischenstufen zwischen den Zuständen "Aus" ohne Lichtemission und "Ein" mit maximaler Intensität einzustellen und unterscheidet sich damit im Sinne der vorliegenden Erfindung von dem nachfolgend beschriebenen Schalter zur digitalen Modulation.

Vorteilhafterweise führt eine synchrone Modulation durch das Steuersignals dazu, dass dadurch die Phase des modulierten Synchronisierungssignal, insbesondere Taktsignals, nicht verändert wird und ein PLL auf der Empfangsseite nicht gestört wird.

In einer denkbaren Ausführungsform des erfindungsgemäßen Magnetresonanztomographen ist der Sender ausgelegt, zur Übertragung des Synchronisierungssignals das optische Datensignal mit einer Modulationsfrequenz ein- und auszuschalten, z.B. mittels Amplitudenumtastung (engl. Amplitude-Shift Keying, ASK), insbesondere On-Off Keying (OOK), wobei der Magnetresonanztomograph ausgelegt ist, zur Übertragung des Steuersignals eine Frequenz des Modulationssignals von einer ersten Modulationsfrequenz zu einer zweiten Modulationsfrequenz ungleich der ersten Modulationsfrequenz zu ändern.

Da hierbei die Intensität des optischen Signals im Takt der Modulationsfrequenz hart umgeschaltet wird, liefert der Lichtsensor ein rechteckförmiges Sensorsignal, dessen Grundfrequenz der ersten bzw. zweiten Modulationsfrequenz entspricht. In Verbindung mit einem vorab beschriebenen Bandpassfilter als Teil des Signalweges für das Sensorsignal oder ein anderes Filter mit frequenzabhängiger Dämpfung wird vorteilhafterweise eine Spektralkomponente (z.B. Harmonische, insbesondere Grundfrequenz) des rechteckförmigen Signals zur weiteren Verarbeitung selektiert.

Die Änderung der Modulationsfrequenz lässt sich bei nichtlinearen Lichtquellen wie z.B. LED oder Halbleiterlaser in der Regel wesentlich einfacher und genauer als eine direkte Steuerung der Leuchtstärke realisieren. Vorzugsweise wird lediglich die Modulationsfrequenz geändert, mit der ein Schalter die Lichtquelle ein- und ausschaltet.

In einer denkbaren Ausführungsform des erfindungsgemäßen Magnetresonanztomographen ist eine Frequenz des Taktsignals ein ungeradzahliges Vielfaches der Modulationsfrequenz. Beispielsweise kann das Taktsignal eine Frequenz von 12.5 MHz haben. Die Modulationsfrequenz wird dann vorzugsweise zwischen einer ersten Modulationsfrequenz und einer zweiten Modulationsfrequenz umgeschaltet, wobei die erste Modulationsfrequenz in dem Beispiel 12.5 MHz betrifft und die zweite Modulationsfrequenz ein Drittel davon, also die Frequenz des Taktsignals gleich die erste Modulationsfrequenz multipliziert mit 1 ist und gleich der zweiten Modulationsfrequenz multipliziert mit 3 ist. Das Umschalten der Modulationsfrequenz erfolgt dabei vorteilhafterweise phasenneutral, also im Zeitraster der Taktfrequenz bzw. ersten Modulationsfrequenz.

Bei einer Modulation des optischen Signals durch Ein- bzw. Ausschalten wird ein Rechtecksignal generiert, das Harmonische auf ungeradzahligen Vielfachen der Modulationsfrequenz aufweist. Ist also die zweite Modulationsfrequenz ein Drittel der Taktsignalfrequenz, so ist die dritte Harmonische bei einem dreifachen der Modulationsfrequenz bzw. genau auf der Frequenz des Taktsignals. Durch ein Bandpassfilter kann dabei die Spektralkomponente bei der Taktsignalfrequenz selektiert und sämtliche weiteren Harmonischen unterdrückt bzw. gedämpft werden, z.B. um mehr als 24 dB, 30 dB oder 36 dB. Es wird also sowohl mit erster Modulationsfrequenz als auch mit zweiter Modulationsfrequenz ein Taktsignal von beispielsweise 12.5 MHz bereitgestellt. Durch das phasenneutrale Umschalten zwischen den beiden Modulationsfrequenzen bleibt dabei auch die Phase des übertragenen Taktsignals unverändert, sodass beispielsweise nachgeschaltete PLL-Schaltungen ein phasenstabiles Ausgangssignal für den Pilotton-Signal-Generator liefern. Gleichzeitig ist aber bei einem gleichanteilfreien, symmetrischen Rechtecksignal die Amplitude der dritten Harmonischen nur ein Drittel so groß wie die Amplitude der Grundwelle (erste Harmonische), wodurch sich bei der Frequenzumtastung eine Amplitudenmodulation des bandpassgefilterten Empfangssignals zwischen einem Drittel und der vollen Amplitude ergibt. Mit dieser Amplitudenmodulation der selektierten Spektralkomponente kann insbesondere ein Steuersignal übertragen werden, wie z.B. zur Steuerung der Pilotton-Signal-Generierung. Bei einem mit dieser Amplitudenmodulation übertragenen digitalen Code können entsprechend unterschiedliche Steuerbefehle übertragen werden, und gleichzeitig ein phasenstabiles Taktsignal bereitgestellt werden, wobei vorzugsweise die Modulation durch einen einfachen Schalter bereitgestellt werden kann.

Ferner wird ein Verfahren zur Bewegungskorrektur einer Magnetresonanzmessung eines Untersuchungsobjekts vorgeschlagen, das umfasst:
- Übertragen eines Synchronisierungssignals des Magnetresonanztomographen an zumindest einen Pilotton-Signal-Generator, insbesondere an eine Empfangseinheit des zumindest einen Pilotton-Signal-Generator,
- Erzeugen und Aussenden eines Pilotton-Signals anhand des Synchronisierungssignals durch den zumindest einen Pilotton-Signal-Generator,
- Empfangen des Pilotton-Signals durch zumindest eine Empfangsspule des Magnetresonanztomographen,
- Durchführen einer Bewegungskorrektur anhand des Pilotton-Signals.

Die Vorteile des Verfahrens zur Bewegungskorrektur einer Magnetresonanzmessung entsprechen im Wesentlichen den Vorteilen des Pilotton-Signal-Generators und des Magnetresonanztomographen, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Das Übertragen des Synchronisierungssignal, das Erzeugen und Aussenden des Pilotton-Signals anhand des Synchronisierungssignals und das Empfangen des Pilotton-Signals erfolgt während der Magnetresonanzmessung. Neben den Pilotton-Signalen werden also auch Magnetresonanzsignale erfasst.

Vorzugsweise wird der Pilotton-Signal-Generator in unmittelbarer Nähe des Herzens oder der Lunge des Untersuchungsobjekts angeordnet. Dadurch kann vorteilhafterweise ein hoher SNR des empfangenen Pilotton-Signal erreicht werden.

Vorzugsweise erfolgt das Übertragen des Synchronisierungssignals des Magnetresonanztomographen an die Empfangseinheit eines Pilotton-Signal-Generators drahtlos, d.h. das Synchronisierungssignal ist ein optisches Datensignal. Vorzugsweise wird das Synchronisierungssignal mit einem, insbesondere ersten, Sensor der Empfangseinheit empfangen und als Ausgangsignal ausgegeben.

Vorzugsweise umfasst das Erzeugen des Pilotton-Signals anhand des Ausgangsignal durch den Pilotton-Signal-Generators einen oder mehrere der folgenden Aspekte:
- Filtern des Ausgangssignals oder eines daraus abgeleiteten Signals mit einem Filter,
- Stabilisieren einer Taktfrequenz eines Oszillators anhand des Ausgangssignals oder eines daraus abgeleiteten Signals,
- Selektieren einer ungeradzahligen Harmonischen des Ausgangssignals oder eines daraus abgeleiteten Signals.

Vorzugsweise wird das Synchronisierungssignals des Magnetresonanztomographen an mehrere Pilotton-Signal-Generatoren übertragen. Vorzugsweise werden die für die jeweiligen Pilotton-Signal-Generatoren generatorspezifischen Informationen mittels eines Zeitmultiplexverfahren übertragen.

Insbesondere erfolgt hierbei ein Identifizieren einer generatorspezifischen Information des Synchronisierungssignals und Erzeugen des jeweiligen Pilotton-Signals in Abhängigkeit der generatorspezifischen Information durch die mehreren Pilotton-Signal-Generatoren.

Vorzugsweise werden die Beiträge der durch die zumindest eine Empfangsspule des Magnetresonanztomographen empfangenen Pilotton-Signale separiert. Vorteilhafterweise kann dadurch die Informationsdichte der Pilotton-Daten drastisch erhöhet werden, so dass eine verbesserte Bewegungskorrektur möglich ist.

Zum Durchführen der Bewegungskorrektur anhand des Pilotton-Signals werden insbesondere bewegungsabhängige Veränderungen des empfangenen Pilotton-Signals mittels einer Signalanalyse in einer Auswerteeinheit des Magnetresonanztomographen erkannt.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: einen Magnetresonanztomographen mit einem Pilotton-Signal-Generator,
- Fig. 2: einen optischen Sender einer ersten Ausführungsform,
- Fig. 3: einen Pilotton-Signal-Generators, der geeignet ist, ein Taktsignal zu verarbeiten,
- Fig. 4: einen optischen Sender einer zweiten Ausführungsform,
- Fig. 5: einen Pilotton-Signal-Generators, der geeignet ist, ein Taktsignal und ein Steuersignal zu verarbeiten,
- Fig. 6: eine Empfangseinheit eines Pilotton-Signal-Generators,
- Fig. 7: einen Kompensationskreis eines Pilotton-Signal-Generators,
- Fig. 8: ein Verfahren Verfahrens zur Bewegungskorrektur einer Magnetresonanzmessung mit Hilfe eines Pilotton-Signal-Generators.

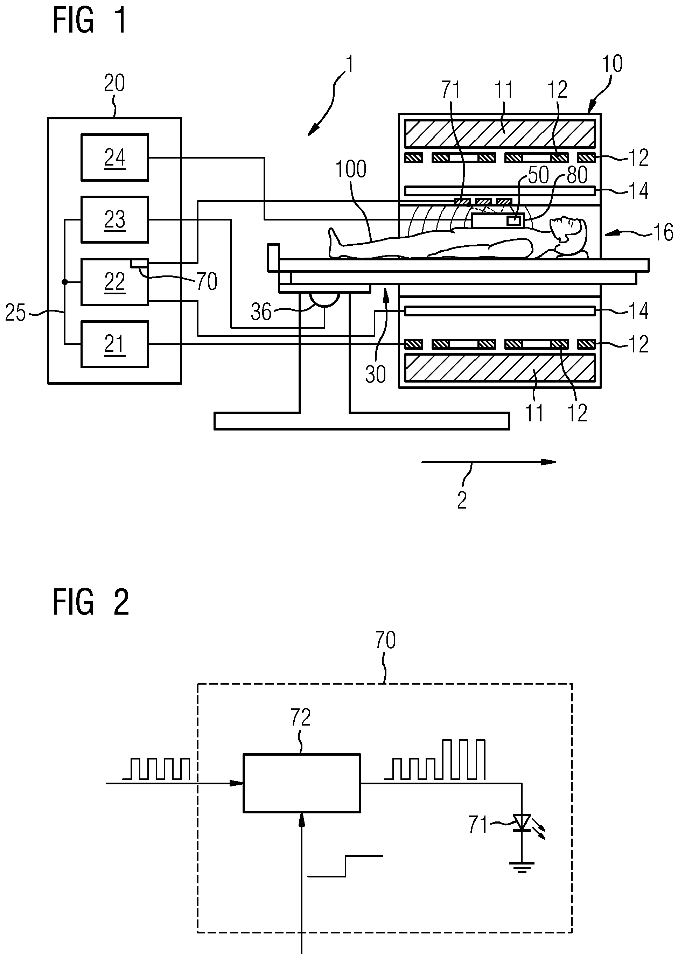

Fig. 1 zeigt eine schematische Darstellung einer Ausführungsform eines Magnetresonanztomographen 1. Der Magnetresonanztomographen 1 umfasst eine Magneteinheit 10, die einen Feldmagneten 11 umfasst, der ein statisches Magnetfeld B0 zur Ausrichtung von Kernspins eine Untersuchungsobjekts, hier eines Patienten 100, in einem Aufnahmebereich des Magnetresonanztomographen 1 erzeugt. Der Aufnahmebereich zeichnet sich durch ein sehr homogenes statisches Magnetfeld B0 aus, wobei die Homogenität insbesondere die Stärke bzw. den Betrag des Magnetfeldes betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Magnetfeldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 zeitlich und räumlich variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen. Ferner ist es denkbar, dass die Körperspule 14 von dem Patient 100 emittierte Magnetresonanzsignale zu empfangen und über eine Signalleitung abgeben kann; in diesem Fall arbeitet die Körperspule 14 als Empfangsspule.

Eine Systemsteuereinheit 20 versorgt die Magneteinheit 10 mit den verschiedenen Signalen für die Gradientenspulen 12 und die Körperspule 14 und wertet die empfangenen Signale aus.

So weist die Systemsteuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Systemsteuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Vorteilhafterweise sind die Empfangsspulen breitbandig genug, so dass sie nicht nur Magnetresonanzsignale empfangen können, sondern auch Pilotton-Signale, die ein anderes Frequenzband aufweisen als die Magnetresonanzsignale.

Auf dem Patienten 100 ist eine Lokalspule 80 mit einem Piloton-Signal-Generator 50 angeordnet. Die arbeitet auch als Empfangsspule, indem sie ein Magnetresonanzsignal aus dem Körper des Patienten 100 aufnimmt und weiterleitet. Die Weiterleitung kann drahtlos erfolgen, vorzugsweise mit einer Funkverbindung.

Vorteilhafterweise sind die Empfangsspulen breitbandig genug, so dass sie nicht nur Magnetresonanzsignale empfangen können, sondern auch Pilotton-Signale, die ein anderes Frequenzband aufweisen als die Magnetresonanzsignale.

Die durch die Lokalspule 80 empfangenen Magnetresonanzsignale und Pilotton-Signale werden an eine Auswerteeinheit 24 der Systemsteuereinheit 20 übertragen, in der insbesondere die Pilotton-Signale ausgewertet werden. Falls sich der Patient 100 während der Magnetresonanzmessung bewegt, werden durch die Bewegung die Pilotton-Signale beeinflusst bzw. geändert. Aus dieser Signaländerung können Rückschlüsse über die Bewegung des Patienten 100 gezogen werden. Beispielsweise können Informationen über die Atmung oder den Herzschlag des Patienten aus Pilotton-Signale abgeleitet werden. Mit Hilfe der Auswertung Pilotton-Signale können somit Bewegungsinformationen ermittelt werden, die zur Bewegungskorrektur eingesetzt werden können.

Die Auswertung der Pilotton-Signale kann verbessert werden, wenn nicht nur die Amplitude der Pilotton-Signale ausgewertet wird, sondern auch ihre Phasenlage. Die Phasenlage kann insbesondere relativ zu einem Referenztakt des Magnetresonanztomographen 1 definiert sein. Die Systemsteuereinheit 20 des Magnetresonanztomographen 1 verfügt intern über entsprechende Taktsignale. Zur Übertragung an den Pilotton-Signal-Generator 50 weist die Systemsteuereinheit 20 weiterhin einen optischen Sender 70 auf, wobei hier mehrere Lichtemitter 71, die das Licht aussenden, dabei im Patiententunnel 16 angeordnet sind. Vorzugsweise weist der Magnetresonanztomograph eine Mehrzahl an Lichtemittern 71 auf, die über eine innere Oberfläche des Patiententunnels 16 verteilt sind, oder eine lichtstreuende innere Oberfläche von außerhalb beleuchten, sodass eine Abschattung eines von den Lichtemittern 71 ausgesandten optischen Signals bei dem Pilotton-Signal-Generator 50 möglichst vermieden wird. Die Lichtemitter 71 können dabei LEDs oder Halbleiterlaser sein, die ein elektrisches Signal von dem optischen Sender 70 zugeführt bekommen und dieses in das optische Signal wandeln und in den Patiententunnel 16 aussenden. Denkbar wäre es aber auch, dass ein LED-Strahler oder Halbleiterlaser in dem optischen Sender 70 bereits eine Wandlung in Licht vornimmt und dieses über Glasfasern und optional optische Splitter zu dem Patiententunnel 16 geführt wird, wobei Glasfaserenden als Lichtemitter 71 in dem Patiententunnel 16 angeordnet sind.

In Fig. 2 ist eine beispielhafte Ausführungsform für einen optischen Sender 70 eines erfindungsgemäßen Magnetresonanztomographen dargestellt. Der optische Sender 70 weist einen Amplitudenmodulator 72 auf. Im einfachsten Fall wird die Amplitude des Signals lediglich mit dem Referenztakt des Referenztakt des Magnetresonanztomographen 1 moduliert.

Optional kann ein hochfrequentes Taktsignal mit einem niederfrequenten Steuersignal moduliert werden. Beispielsweise kann der Amplitudenmodulator 72 einen Multiplizierer aufweisen, der das Taktsignal mit einem niederfrequenten Steuersignal multipliziert. Vorzugsweise erfolgt eine Modulation des Taktsignals phasensynchron, d.h. mit ansteigender oder fallender Flanke des Taktsignals. Dies kann beispielsweise erreicht werden, in dem das Steuersignal über ein Sample- und Hold-Glied an den Multiplizierer durchgeschaltet wird, wobei das Sample-und-Hold-Glied von dem Taktsignal gesteuert wird, z.B. bei einem Low-Pegel des Taktsignals durchschaltet. Ein Ausgangssignal des Amplitudenmodulators 72 wird direkt oder über eine Endstufe an eine oder mehrere LED bzw. LED-Strahler oder Halbleiterlaser zur Wandlung in ein optisches Signal ausgegeben.

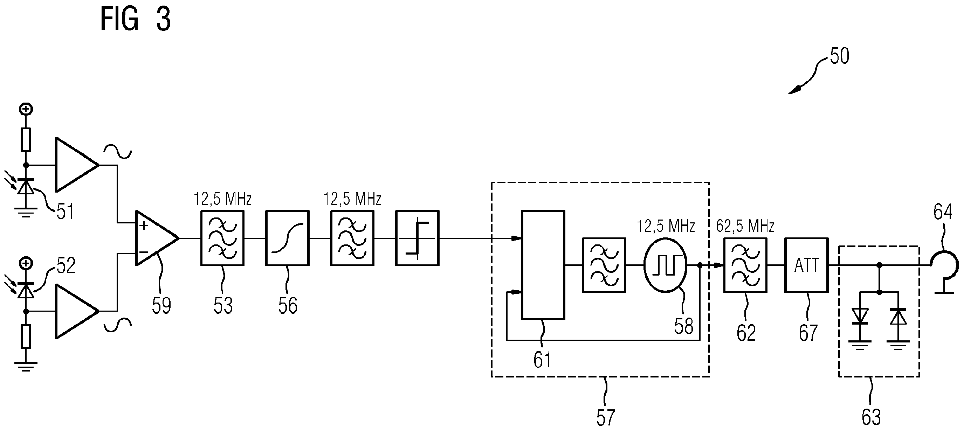

In Fig. 3 sind in einer beispielhaften Ausführungsform Komponenten des Pilotton-Signal-Generators 50 dargestellt, die an einer Übertragung bzw. Rückgewinnung des Taktsignals und Steuersignals beteiligt sind. Andere Elemente des Pilotton-Signal-Generators 50 sind aus Gründen der Übersichtlichkeit nicht dargestellt.

Ein erster optischer Sensor 51, beispielsweise eine Fotodiode mit einem Vorverstärker, wandelt das optische Signal in ein elektrisches Signal um. Ein Filter 53, beispielsweise ein Bandpassfilter oder Tiefpassfilter, lässt bevorzugt ein Signal mit einer Frequenz des Taktsignals durch und dämpft Signale mit anderen Frequenzen beispielsweise um mehr als 24 dB, 30 dB, 36 dB.

Das gefilterte Signal wird weiteren Komponenten zugeführt, um einen stabilen Mastertakt für die den Pilotton-Signal-Generator 50 zu erzeugen. Das gefilterte Signal des ersten Sensors 51 wird dabei zunächst in einem begrenzenden Verstärker 56 so verstärkt, dass die Amplitudenschwankungen durch die Amplitudenbegrenzung beseitigt werden und nur noch die Phaseninformation der Trägerwelle verbleiben. In einem PLL-Regelkreis wird damit die Frequenz und die Phase eines Oszillators 58, insbesondere eines spannungsgesteuerten Quartzoszillators, stabilisiert. Das Ausgangssignal des Oszillators 58 oder ein daraus abgeleitetes Signal ist der Mastertakt für den Pilotton-Signal-Generator 50.

Ein Filter 62 selektiert eine ungeradzahlige Harmonische eines Ausgangssignals des Phase-Lock-Loop-Schaltkreises 57.

Beispielsweise wird die fünfte Harmonische von 62.5 MHz einer Grundschwingung von 12.5 Mhz gewählt.

Der Pilotton-Signal-Generator 50 umfasst ferner eine Sendeantenne 64 zum Aussenden des Pilotton-Signals. In diesem Beispiel bildet die Sendeantenne 64 mit einem Entkopplungselement 63 einen Resonanzkreis. Das Entkopplungselement 63 umfasst hier zwei antiparallel geschaltete Dioden, die vor der Sendeantenne 64 nach Masse geschaltet sind. Damit wird sichergestellt, dass eine von einem Anregungspuls des Magnetresonanztomographen 1 in die Sendeantenne 64 induzierte Spannung unterhalb einer Durchlassspannung begrenzt wird.

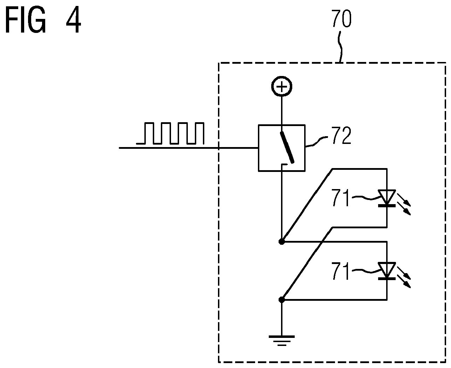

In Fig. 4 ist eine andere mögliche Ausführungsform für den optischen Sender 70 dargestellt. Diese Ausführungsform beruht auf dem Gedanken, dass Rechtecksignale Oberwellenanteile aufweisen, deren Frequenz einem ungeradzahligen Vielfachen der Grundfrequenz entsprechen. Beispielhaft dargestellt sind hier als Eingangssignale des optischen Senders ein Rechtecksignal mit hoher Frequenz, auch als erste Modulationsfrequenz bezeichnet, beispielsweise von 12.5 MHz, und ein Rechtecksignal mit niedrigerer Frequenz bzw. zweiter Modulationsfrequenz von (12.5/3) MHz. Die Systemsteuereinheit 20 stellt diese Signale mit hoher Genauigkeit abgeleitet von einem stabilen Mastertakt bereit. Auch wenn der optische Sender 70 also ein optisches Signal mit der zweiten Modulationsfrequenz abstrahlt, weist dieses dann Frequenzanteile mit der ersten Modulationsfrequenz von 12.5 MHz auf.

Von dem ersten Modulationssignal oder dem zweiten Modulationssignal getaktet schaltet der optische Sender mittels eines elektronischen Schalters die Stromversorgung für die LED bzw. einen Halbleiterlaser als Lichtemitter 71 bzw. Lichtquelle ein und aus und erzeugt so ein mit dem Rechtecksignal moduliertes optisches Signal. Vorzugsweise werden eine Vielzahl von über den Patiententunnel 16 verteilten LED gleichzeitig geschaltet, um eine Abschattung des Sensors 51 zu vermeiden. Alternativ kann auf vorteilhafte Weise die Innenwand des Tunnels lichtstreuend ausgestaltet werden und von außerhalb des Tunnels angestrahlt werden. Auf vorteilhafte Weise ist eine Modulation mit einem Rechtecksignal durch einen Schalter einfach zu realisieren und effizienter als eine lineare Intensitätsmodulation.

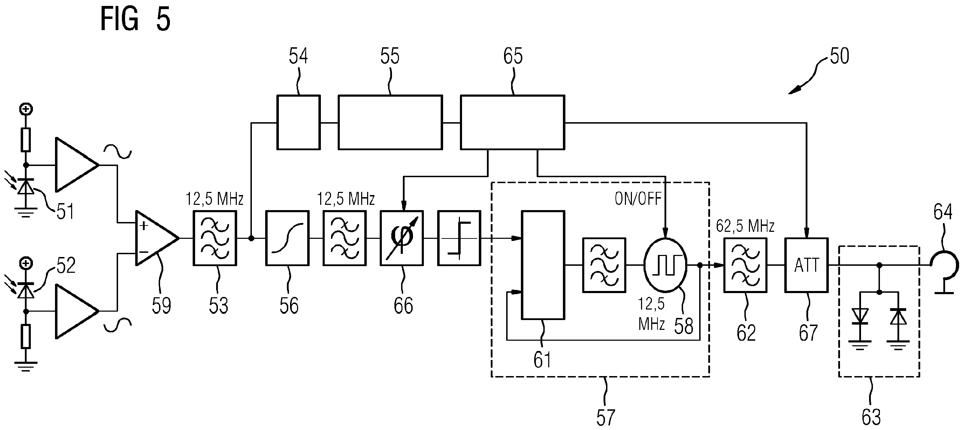

Durch den Wechsel zwischen erster Modulationsfrequenz und zweiter Modulationsfrequenz steht dabei immer auch wegen der Oberwellen ein Signalanteil mit der ersten Modulationsfrequenz bereit, der durch das Filter 53 in dem Pilotton-Signal-Generator selektiert wird. Da die Oberwellenanteile immer eine geringere Amplitude als die Grundwelle aufweisen, führt der Wechsel zwischen den Modulationsfrequenzen zu einer Amplitudenmodulation in einem Spektralbereich der ersten Modulationsfrequenz, die beispielsweise mit dem Pilotton-Signal-Generator 50 aus Fig. 5 ausgewertet werden kann. Sind die Signale von erster Modulationsfrequenz und zweiter Modulationsfrequenz in phasenstabiler Beziehung, vorzugsweise indem die Flanken des Signals mit der zweiten Modulationsfrequenz synchron zu Flanken des Signals mit der ersten Modulationsfrequenz sind, so wird das über eine PLL erzeugte Taktsignal durch die Frequenz-Umtastung nicht gestört Dies kann beispielsweise erreicht werden, indem die zweite Modulationsfrequenz durch phasensynchrone Frequenzteilung aus der ersten Modulationsfrequenz gewonnen wird.

In Fig. 5 ist eine gegenüber Fig. 3 erweiterte Ausführungsform des Pilotton-Signal-Generators dargestellt. Hier wird das gefilterte Signal in einem Demodulationszweig einem Amplitudendemodulator 54 zugeführt, um ein auf das Taktsignal aufmoduliertes Steuersignal zurückzugewinnen. Im einfachsten Fall kann der Amplitudendemodulator 54 aus einer Diode als Gleichrichter und einem Tiefpass bzw. Pufferkondensator bestehen.

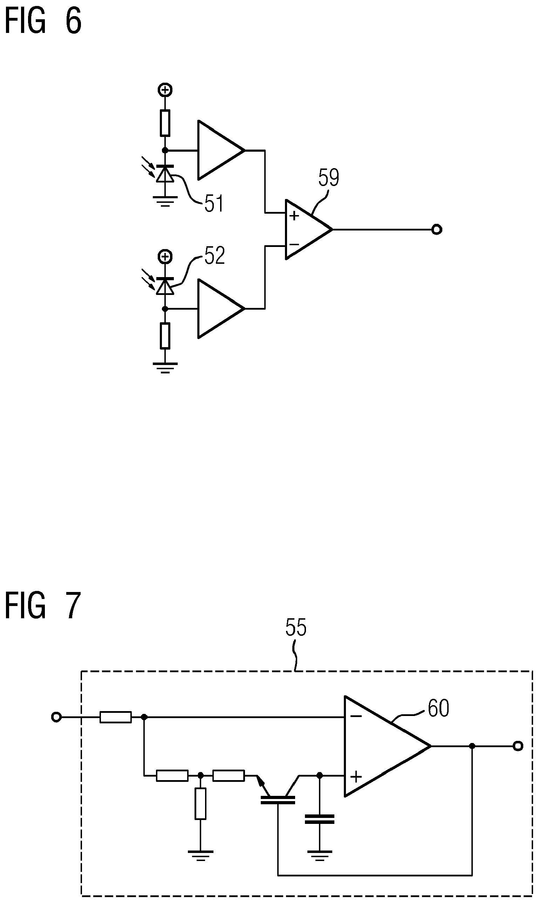

Oftmals weist das demodulierte Signal immer noch Schwankungen auf, die z.B. durch wechselnde Abschattungen auf dem Ausbreitungs-weg im Patiententunnel verursacht werden. Diese Schwankungen können werden mit einem Kompensationskreis 55 ausgeglichen, der zu einer nachfolgenden Fig. 7 erläutert wird.

Mit einer Steuerkomponente 65 kann anhand des Steuersignals des Synchronisierungssignals eine Phasensteuerelement 66 und/oder ein Amplitudensteuerelement 67 gesteuert werden. Mittels des Steuersignals kann somit die Generierung des Pilotton-Signals gesteuert werden. Insbesondere kann mit dem Amplitudensteuerelement 67 die Amplitude und mit Phasensteuerelement 66 die Phase kontrolliert werden. Beispielsweise kann bei Detektion schneller Bewegung, z.B. Herzbewegung, mit hoher Pilotton-Amplitude gesendet werden, bei Detektion langsamer Bewegung, z.B. Atmung, mit einer niedrigen; bei Magnetresonanzmessungen, die nicht bewegungsempfindlich sind, könnte das Pilotton-Signal abschaltet werden.

Optional umfasst die Steuerkomponente 65 einen ID-Decoder, der ausgebildet ist, aus dem Steuersignal für den Pilotton-Signal-Generator 50 generatorspezifische Information, insbesondere eine Identifizierungsinformation, zu ermitteln. Dies ist vor allem dann vorteilhaft, wenn mehrere Pilotton-Signal-Generatoren bei einer Magnetresonanzmessung eingesetzt werden.

Anhand der für den Pilotton-Signal-Generator 50 generatorspezifische Information kann ein spezifisches Einstellen der relativen Phasen und Amplituden ermöglichen, um beispielsweise die "Ausleuchtung" des Patienten 100 mit dem Pilotton-Signal zu optimieren. Zusätzlich könnten die Amplituden und Phasen auf einer Zeitskala, die schneller als die zu detektierende Bewegung ist, geschaltet werden. Durch dieses der Auswerteeinheit 24 bekannte Zeitmultiplexverfahren, insbesondere "time/phase multiplexing", wäre es möglich, die Beiträge der einzelnen Pilotton-Signal-Generatoren zum Gesamtsignal zu separieren und die Informationsdichte der Pilotton-Daten deutlich zu erhöhen.
Fig. 6 zeigt schematisch eine vorteilhafte Kombination eines ersten Sensors 51 und eines zweiten Sensors 52 zum Detektieren des optischen Datensignals. Die Kombination reduziert insbesondere elektromagnetische Störungen auf den Empfang des optischen Datensignals, die durch den Betrieb des Magnetresonanztomographen 1 verursacht werden.

Zu diesem Zweck sind der erste Sensor 51 und der zweite Sensor 52 benachbart zueinander angeordnet, um eine Induktion in Verbindungsleitungen zu minimieren und beide Sensoren 51, 52 möglichst gleichen elektromagnetischen Feldern auszusetzen. Der Abstand ist vorzugsweise kleiner als 2 cm, 1 cm oder 5 mm.

Der erste Sensor 51, hier eine Fotodiode, ist dabei in Fig. 6 unmittelbar mit der positiven Versorgungsspannung in Sperrrichtung verbunden, während die Verbindung zum Massepotential über einen Widerstand erfolgt.

Für den zweiten Sensor 52 sind die Rollen von Widerstand und Sensor vertauscht, der Widerstand ist also direkt mit der positiven Versorgungsspannung verbunden und der zweite Sensor 52 mit dem Massepotential. Durch das Vertauschen der Anordnung erzeugt ein gleiches optisches Signal in den beiden Sensoren 51, 52 ein elektrisches Signal mit vergleichbarer Amplitude, aber entgegengesetztem Vorzeichen.

Das von den Sensoren 51, 52 erzeugte elektrische Signal wird verstärkt und einem Differenzverstärker 59 am invertierenden bzw. am nicht-invertierenden Eingang zugeführt. Auf vorteilhafte Weise addieren sich so aufgrund des unterschiedlichen Vorzeichens die von dem optischen Signal an den Sensoren 51, 52 verursachten elektrischen Signale am Ausgang des Differenzverstärkers 59. Elektromagnetische Störungen hingegen induzieren vorzugsweise Störsignale mit gleichem Vorzeichen in beiden Zweigen, sodass sich diese in dem Differenzverstärker 59 im Wesentlichen Aufheben. Gleichzeitig ist das Signal-zu-Rauschverhältnis des Summensignals um 3dB größer als das der Einzelsignale, da die Rauschbeiträge der elektrischen Komponenten unkorreliert sind und sich somit leistungsmäßig addieren, während sich die korrelierten Empfangssignale spannungsmäßig addieren.

In Fig. 7 ist eine mögliche Ausführungsform eines Kompensationskreises 55 schematisch dargestellt. Das gefilterte und demodulierte Sensorsignal wird dem invertierenden Eingang eines Komparators 60 zugeführt. Gleichzeitig wir das Sensorsignal durch einen resistiven Spannungsteiler reduziert, über ein RC-Glied tiefpassgefiltert und dem nicht-invertierenden Eingang des Komparators 60 zugeführt. Die Kapazität des RC-Gliedes dient gleichzeitig als Ladekapazität eines Track-and-Hold Gliedes, dessen elektrischer Schalter hier als MOS-FET ausgeführt ist. Das Track-und-Hold-Glied wird dabei durch den Ausgang des Komparators 60 angesteuert, sodass der Schalter des Track-und-Hold-Gliedes geöffnet wird, wenn das SensorSignal kleiner als das Referenz-Signal wird. Der Komparator kann beispielsweise als Differenzverstärker mit hoher Verstärkung oder einem anderen äquivalenten Schaltkreis realisiert sein.

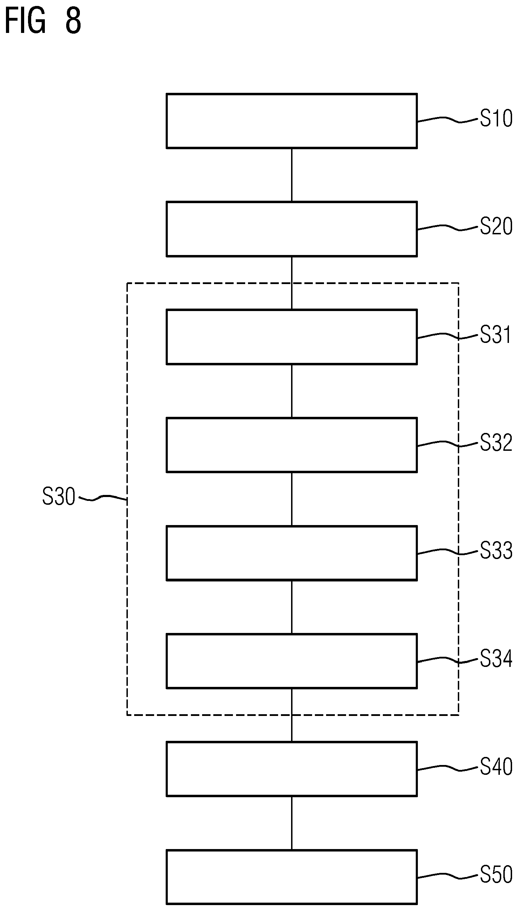

In Fig. 8 wird ein Verfahren zur Bewegungskorrektur einer Magnetresonanzmessung eines Untersuchungsobjekts vorgeschlagen, das umfasst:
In S10 wird ein Untersuchungsobjekt, insbesondere ein Patient (100), in einem Magnetresonanztomographen. In S20 wird ein Synchronisierungssignals des Magnetresonanztomographen (1) an zumindest einen Pilotton-Signal-Generator (50), insbesondere an eine Empfangseinheit des zumindest einen Pilotton-Signal-Generator, übertragen.

In S30 wird ein Pilotton-Signal anhand des Synchronisierungssignals durch den zumindest einen Pilot-ton-Signal-Generator (50) erzeugt und ausgesendet. Beim Erzeugen des Pilotton-Signals wird in S31 das Ausgangssignals und/oder ein daraus abgeleitetes Signal mit einem Filter gefiltert. In S32 wird eine generatorspezifische Information des Synchronisierungssignals und Erzeugen des jeweiligen Pilotton-Signals in Abhängigkeit der generatorspezifischen Information durch die mehreren Pilotton-Signal-Generatoren identifiziert. In S33 wird eine Taktfrequenz eines Oszillators anhand des Ausgangssignals oder eines daraus abgeleiteten Signals stabilisiert. In S34 wird eine ungeradzahlige Harmonischen des Ausgangssignals oder eines daraus abgeleiteten Signals selektiert.

Es ist denkbar, dass die Schritte S31-S34 auch in einer anderen Reihenfolge, parallel und/oder wiederholt durchgeführt werden.

In S40 wird das Pilotton-Signal durch zumindest eine Empfangsspule des Magnetresonanztomographen empfangen. In S50 wird eine Bewegungskorrektur anhand des Pilotton-Signals durchgeführt.

Es wird abschließend noch einmal darauf hingewiesen, dass es sich bei den vorhergehend detailliert beschriebenen Verfahren sowie bei dem dargestellten Pilotton-Signal-Generator und Magnetresonanztomograph lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Pilotton-Signal-Generator,
wobei der Pilotton-Signal-Generator (50) eine Empfangseinheit zum Empfang eines Synchronisierungssignals einer Systemsteuereinheit (20) eines Magnetresonanztomographen (1) umfasst,
wobei das Synchronisierungssignals ein Taktsignal umfasst,
wobei der Pilotton-Signal-Generator (50) ausgebildet ist, ein Pilotton-Signal in Abhängigkeit von dem Synchronisierungssignal auszusenden.

2. Pilotton-Signal-Generator nach Anspruch 1,
wobei das Synchronisierungssignal ein optisches Datensignal ist,
wobei die Empfangseinheit einen ersten Sensor (51) zum Empfang des optischen Datensignals aus der Umgebung des Pilotton-Signal-Generators (50) umfasst und ausgebildet ist, aus dem optischen Datensignal ein elektrisches Sensorsignal zu erzeugen.

3. Pilotton-Signal-Generator nach Anspruch 2,
wobei die Empfangseinheit einen zu dem ersten Sensor (51) benachbarten zweiten Sensor (52) umfasst,
wobei der erste Sensor (51) ausgelegt ist, aus dem optischen Datensignal ein erstes Ausgangssignal zu erzeugen und der zweite Sensor (52) ausgelegt ist, aus dem gleichen optischen Datensignal ein zweites Ausgangssignal zu erzeugen,
wobei das erste Ausgangssignal eine zu dem zweiten Ausgangssignal inverse Amplitude aufweist,
wobei die Pilotton-Signal-Generator (50) einen Inverter aufweist, der das Ausgangssignal des ersten Sensors (51) invertiert, und ein Summierglied, das ausgelegt ist, das invertierte Ausgangssignal des ersten Sensors und das Ausgangssignal des zweiten Sensors (52) zu dem Sensorsignal zu addieren.

4. Pilotton-Signal-Generator nach Anspruch 2 oder 3,
wobei der Pilotton-Signal-Generator (50) einen Phase-Lock-Loop-Schaltkreis (57) zur Synchronisation des Pilotton-Signals umfasst,
wobei ein aus dem Sensorsignal abgeleitetes Signal als Referenzsignal für den Phase-Lock-Loop-Schaltkreis (57) dient.

5. Pilotton-Signal-Generator nach Anspruch 4,
wobei der Pilotton-Signal-Generator (50) ein Filter (53) aufweist, das ausgelegt ist, eine Modulationsfrequenz des Sensorsignals zu selektieren,
wobei der Phase-Lock-Loop-Schaltkreis (57) ausgelegt ist, einen Oszillator (58) in Abhängigkeit von der Modulationsfrequenz, und vorzugsweise der Modulationsphase, zu stabilisieren.

6. Pilotton-Signal-Generator nach einem der Ansprüche 4 oder 5,
wobei der Pilotton-Signal-Generator (50) ein Filter (62) aufweist, das ausgelegt ist, eine ungeradzahlige Harmonische eines Ausgangssignals des Phase-Lock-Loop-Schaltkreises (57) zu selektieren.

7. Pilotton-Signal-Generator nach einem der Ansprüche 2 bis 6,
wobei der Pilotton-Signal-Generator (50) einen Amplitudendemodulator (54) mit einem Kompensationskreis (55) umfasst, der ausgelegt ist, einen im Vergleich zu einer Modulationsfrequenz des Sensorsignals niederfrequenten Signalanteil des Sensorsignals zu kompensieren.

8. Pilotton-Signal-Generator nach einem der vorangehenden Ansprüche,
wobei das Synchronisierungssignal ein Steuersignal umfasst,
wobei der Pilotton-Signal-Generator (50) eine Steuerkomponente (65) umfasst, die geeignet ist, anhand des Steuersignals eine Amplitude und/oder Phase des Pilotton-Signals zu steuern.

9. Pilotton-Signal-Generator nach Anspruch 8,
wobei die Steuerkomponente (65) des Pilotton-Signal-Generators (50) geeignet ist, anhand des Steuersignals eine für den Pilotton-Signal-Generator (50) generatorspezifische Information zu ermitteln, um den Pilotton-Signal-Generator spezifisch zu steuern.

10. Magnetresonanztomograph zur Untersuchung eines Untersuchungsobjekts (100),
wobei der Magnetresonanztomograph (1) einen optischen Sender (70) umfasst,
wobei der optische Sender (70) ausgelegt ist, ein optisches Datensignal, insbesondere Synchronisierungssignal, mittels einer optischen Freiluftübertragung an zumindest einen Pilotton-Signal-Generator (50) zu übertragen.

11. Magnetresonanztomograph nach Anspruch 10,
wobei der Magnetresonanztomograph (1) zumindest eine Empfangsspule (80) umfasst,
wobei die zumindest eine Empfangsspule (80) ausgebildet ist, durch den zumindest einen Pilotton-Signal-Generator (50) ausgesendete Pilotton-Signale zu empfangen.

12. Magnetresonanztomograph nach Anspruch 10 oder 11,
wobei die Auswerteeinheit (24) ausgebildet ist, empfangene Pilotton-Signale mehrerer Pilotton-Signal-Generatoren zu separieren.

13. Magnetresonanztomograph nach einem der Ansprüche 10 bis 12,
wobei der optische Sender (70) ausgelegt ist, das optische Datensignal in den Patiententunnel zu strahlen und/oder durch Streuung an einer Oberfläche des Patiententunnels zu verteilen.

14. Verfahren zur Bewegungskorrektur einer Magnetresonanzmessung vorgeschlagen, das umfasst:
- Übertragen eines Synchronisierungssignals des Magnetresonanztomographen an eine Empfangseinheit eines Pilotton-Signal-Generators (S20),
- Erzeugen und Aussenden eines Pilotton-Signals anhand des Synchronisierungssignals durch den Pilotton-Signal-Generators (S30),
- Empfangen des Pilotton-Signals durch zumindest eine Empfangsspule des Magnetresonanztomographen (S40),
- Durchführen einer Bewegungskorrektur anhand des Pilotton-Signals (S50).

15. Computerprogrammprodukt, welches ein Programm umfasst und direkt in einen Speicher einer programmierbaren Systemsteuereinheit (20) eines Computertomographen (1) ladbar ist, mit Programmmitteln, um ein Verfahren nach Anspruch 14 auszuführen, wenn das Programm in der Systemsteuereinheit (20) des Computertomographen (1) ausgeführt wird.
